# EUROPÄISCHE PATENTSCHRIFT

(11) **EP 1 330 192 B1**
(45) Veröffentlichungstag und Bekanntmachung des Hinweises auf die Patenterteilung: **09.06.2004**
(21) Anmeldenummer: 01986859.5
(22) Anmeldetag: 07.12.2001
(51) Int. Cl.: A61B 17/16

(54) **BOHRWERKZEUG FÜR EINE CHIRURGISCHE BOHRMASCHINE**
DRILLING TOOL FOR A SURGICAL DRILL
OUTIL DE PERCAGE POUR UNE PERCEUSE CHIRURGICALE

(30) Priorität: 23.12.2000 DE 10064975
(43) Veröffentlichungstag der Anmeldung: 30.07.2003
(73) Patentinhaber: Aesculap AG & Co. KG, 78532 Tuttlingen (DE)
(72) Erfinder: HERMANN, Reiner, 78567 Fridingen (DE)
(74) Vertreter: Böhme, Ulrich, Dr. Dipl.-Phys.
(86) Internationale Anmeldenummer: PCT/EP2001/014424
(87) Internationale Veröffentlichungsnummer: WO 2002/051319

(56) Entgegenhaltungen:
- DE-U- 20 021 903
- US-A- 3 128 768
- US-A- 3 384 085
- US-A- 5 667 509

## Beschreibung

Die Erfindung betrifft ein Bohrwerkzeug für eine chirurgische Bohrmaschine mit einem einen Schaft, eine Spitze und ein Kupplungsteil zur Herstellung einer Drehverbindung mit einem Rotationsantrieb der Bohrmaschine aufweisenden Bohrer, auf dessen Schaft eine diesen umgebende proximale Schutzhülse mit einem proximalen und einem distalen Ende gehalten ist, in welche federnd eine den Bohrer zwischen deren distalem Ende und der Spitze des Bohrers zumindest über einen Teil seiner Länge umgebende distale Schutzhülse einschiebbar ist.

Derartige Bohrer können sehr unterschiedlich ausgebildet sein, es kann sich dabei um herkömmliche Spiralbohrer handeln, um Fingerfräser, um Hohlbohrer, um Spickdrähte oder um andere Werkzeuge, die durch Drehbewegung in den Knochen eingeführt werden.

In allen Fällen besteht beim Einsatz derartiger Bohrwerkzeuge am menschlichen Körper die Gefahr, daß umliegendes Gewebe durch den rotierenden Bohrer verletzt wird.

Es ist bereits bekannt, zu diesem Zweck den Bohrer mit einer Schutzhülse zu umgeben, die federnd in die Bohrmaschine eingeschoben werden kann (WO-A-9 724 991). Allerdings führt diese Konstruktion dazu, daß die Bohrmaschine ganz erheblich aufwendiger konstruiert werden muß, da in ihr ein den Motor umgebender Raum vorhanden sein muß, in dem die Schutzhülse aufgenommen werden kann. Außerdem ist es auf diese Weise nicht möglich, die Dimension der Schutzhülse an die Dimension des jeweilig verwendeten Bohrers anzupassen, und daher hat sich diese Konstruktion insgesamt nicht durchgesetzt.

In der US-Patentschrift 3,128,768 ist ein chirurgischer Bohrer beschrieben, bei dem der Bohrer von einer Schutzhülse umgeben wird, diese ist jedoch am Bohrer festgelegt, so daß sich die Gewebeschutzhülse mit dem Bohrer mitdreht. Lediglich ein teleskopierender Abschnitt der Gewebeschutzhülse ist gegenüber dem festgelegten Teil frei drehbar und kann sich mittels spitzer Stifte am Gewebe abstützen, so daß ein Mitdrehen dieses Abschnittes verhindert wird. Dies ist eine komplizierte Konstruktion, und durch die Drehung der Gewebeschutzhülse ist die Verletzungsgefahr nicht vollständig ausgeschlossen.

Ausgehend von diesem Stand der Technik ist es Aufgabe der Erfindung, ein Bohrwerkzeug der gattungsgemäßen Art so auszubilden, daß mit ihm Verletzungen des umgebenden Gewebes nicht zu befürchten sind.

Diese Aufgabe wird bei einem Bohrwerkzeug der eingangs beschriebenen Art erfindungsgemäß dadurch gelöst, daß die proximale Schutzhülse eine Drehsicherung aufweist, die bei in die Bohrmaschine eingesetztem Bohrer eine Drehung der proximalen Schutzhülse relativ zur Bohrmaschine verhindert.

Die Schutzvorrichtung ist dabei jeweils an die Dimension des Bohrers angepaßt, da die Schutzvorrichtung für einen bestimmten Bohrer vorgesehen ist, eine besondere Größenanpassung der Bohrmaschine ist dazu nicht notwendig. Insbesondere kann ein solches Bohrwerkzeug in herkömmlicher Weise einhändig in eine Bohrmaschine eingesetzt werden, und auch die Bohrmaschine kann einhändig bedient werden, dabei wird der Bohrer beim Einsatz vollständig nach außen hin abgedeckt, und beim Eindringen des Bohrers in das zu bearbeitende Material verschiebt sich die distale Schutzhülse so, daß auch der Bohrvorgang selbst einhändig erfolgen kann. Die Schutzhülse wird dabei auch in ihrem proximalen Teil durch die Drehsicherung festgehalten, so daß die Schutzhülse insgesamt nicht verdreht wird, dadurch wird die Verletzungsgefahr ausgeschlossen.

Günstig ist es, wenn die distale Schutzhülse im ausgeschobenen Zustand den Bohrer bis über dessen Spitze abdeckt, so daß kein unbedeckter Bereich des Bohrers verbleibt, der Verletzungen verursachen könnte.

Es ist günstig, wenn die proximale Schutzhülse auf dem Schaft des Bohrers um dessen Längsachse verdrehbar gehalten ist. Insbesondere ist es vorteilhaft, wenn die proximale Schutzhülse und der Schaft des Bohrers zueinander hin offene Umfangsnuten aufweisen, in die mindestens ein gemeinsames Lagerelement eingreift. Dadurch ist eine axiale Festlegung der beiden Teile gegeneinander unter Beibehaltung einer Drehverbindungsmöglichkeit gegeben.

Das Lagerelement kann insbesondere elastisch verformbar sein und beim axialen Verschieben der proximalen Schutzhülse relativ zum Schaft elastisch in eine der beiden Umfangsnuten einschnappen. Dadurch können die proximale Schutzhülse und der Schaft in einfachster Weise dadurch verbunden werden, daß sie axial gegeneinander verschoben werden; sobald die beiden Umfangsnuten einander gegenüberliegen, schnappt das elastische Lagerelement in beide ein und fixiert die beiden Teile relativ zueinander, diese Verbindung ist allerdings unter Überwindung einer bestimmten Axialkraft auch wieder lösbar, so daß die gesamte Schutzvorrichtung ohne weiteres vom Bohrer abgezogen werden kann.

Dabei ist es vorteilhaft, wenn eine Umfangsnut eine sehr geringe Tiefe aufweist und die andere eine größere Tiefe. Die Umfangsnut mit der größeren Tiefe nimmt dabei dauerhaft das Lagerelement auf, das vorzugsweise als Ring ausgebildet ist, und dieses Lagerelement schnappt dann in die weniger tiefe Umfangsnut ein zur Ausbildung einer auch wieder lösbaren Axialfestlegung.

Beispielsweise kann die Drehsicherung durch einen radialen Vorsprung der proximalen Schutzhülse gebildet sein, der in einen Rücksprung der Bohrmaschine eingreift.

Es ist auch vorteilhaft, wenn die proximale Schutzhülse in der Bohrmaschine in axialer Richtung festlegbar ist, so daß während des Einsatzes sichergestellt ist, daß die proximale Schutzhülse nicht vom Bohrer abgeschoben werden kann.

Diese Festlegung kann beispielsweise dadurch erfolgen, daß die proximale Schutzhülse mindestens einen Rücksprung aufweist, in den ein Riegelvorsprung der Bohrmaschine einschiebbar ist.

Bei einer besonders bevorzugten Ausführungsform ist vorgesehen, daß die proximale Schutzhülse einen Anschlag trägt, mit dem sie eine Verschiebung des Bohrers in distaler Richtung verhindert. Damit kann die proximale Schutzhülse zusätzlich die axiale Festlegung des Bohrers in der Bohrmaschine übernehmen, wenn die proximale Schutzhülse in der beschriebenen Weise in axialer Richtung in der Bohrmaschine festgelegt ist, verhindert sie mittels dieses Anschlages eine axiale Verschiebung des Bohrers aus der Bohrmaschine heraus.

Dabei ist es vorteilhaft, wenn das Kupplungsteil in axialer Richtung formschlüssig in eine Kupplungsaufnahme der Bohrmaschine eingreift. Diese Kupplung wird dann dadurch aufrechterhalten, daß eine axiale Verschiebung des Bohrers relativ zu Bohrmaschine verhindert wird, im beschriebenen Beispiel durch die axiale Festlegung der proximalen Schutzhülse und den Anschlag der Schutzhülse am Bohrer.

Bei einer besonders bevorzugten Ausführungsform der Erfindung ist vorgesehen, daß der Bohrer an seinem Schaft einen Bund trägt mit einer Kante, die an einer Stufe der proximalen Schutzhülse anliegt, der eine Umfangsnut zur Aufnahme eines O-Ringes aufweist, wobei der O-Ring in eine gegenüberliegende Umfangsnut der proximalen Schutzhülse eingreift, und der stirnseitig einen Mitnehmer trägt, der als Kupplungsteil in eine Mitnahmeöffnung des Rotationsantriebes der Bohrmaschine einführbar ist. Der Bund kann dabei einteilig mit dem Schaft ausgebildet sein, es ist auch möglich, daß der Bund ein Kunststoffteil ist, das auf den Bohrerschaft aufgebracht und dort befestigt ist, beispielsweise durch Umspritzen des Bohrerschaftes.

Weiterhin ist es vorteilhaft, wenn der Schaft von einer Schraubenfeder umgeben ist, die sich einerseits am Schaft und andererseits an der distalen Schutzhülse abstützt.

Es ist dabei günstig, wenn die Schraubenfeder mindestens an einem Ende eine quer zur ihrer Längsachse verlaufende Endwindung aufweist, mit der sie an einer Abstützfläche des Schaftes beziehungsweise der distalen Schutzhülse anliegt. Dadurch ist eine Verdrehung der Schraubenfeder gegenüber den Stützflächen möglich, und zwar gegebenenfalls in beiden Richtungen.

Die Schraubenfeder kann bei einer besonderen Ausführungsform auch drehfest mit der proximalen Schutzhülse verbunden sein, beispielsweise durch Einspritzen in ein Kunststoffteil des Bohrers.

Günstig ist es, wenn die distale Schutzhülse gegenüber der proximalen Schutzhülse um deren Längsachse unverdrehbar ist. Die beiden Schutzhülsen bilden somit eine nur gemeinsam verdrehbare Einheit, und wenn die proximale Schutzhülse gegen eine Verdrehung gegenüber der Bohrmaschine gesichert ist, gilt dies auch für die distale Schutzhülse. Insgesamt wird somit bei dieser Ausgestaltung der Bohrer von einer Schutzeinrichtung umgeben, die nicht mit dem Bohrer umläuft und die daher maximale Sicherheit in der Umgebung des Bohrers bietet.

Um dies zu erreichen, kann beispielsweise vorgesehen sein, daß die distale Schutzhülse einen unrunden Querschnitt aufweist und in eine komplementäre Öffnung der proximalen Schutzhülse eintaucht. Der unrunde Querschnitt kann beispielsweise dadurch erzeugt werden, daß die distale Schutzhülse bei einem kreisförmigen Querschnitt mindestes einseitig abgeflacht ist.

Günstig ist es, wenn die distale Schutzhülse eine Tiefenskala trägt, der Operateur kann dann an dieser Tiefenskala feststellen, wie weit die distale Schutzhülse in die proximale Schutzhülse eingeschoben worden ist, und dies ist ein Maß für den Vorschub des Bohrers in dem zu bearbeitenden Material, da sich die distale Schutzhülse an der Oberfläche dieses Materials abstützt.

Bei einer besonders bevorzugten Ausführungsform ist vorgesehen, daß auf der distalen Schutzhülse ein Schleppring angeordnet ist, der dort im Reibsitz gehalten und in axialer Richtung unter Überwindung der Reibkraft verschiebbar ist. Beim Eintauchen der distalen Schutzhülse in die proximale Schutzhülse wird dieser Schleppring verschoben und zeigt nach dem federnden Ausschieben der distalen Schutzhülse aus der proximalen Schutzhülse an, wie weit die distale Schutzhülse maximal in die proximale Schutzhülse eingetaucht ist, daran kann man die erreichte Bohrtiefe ablesen.

Die nachfolgende Beschreibung bevorzugter Ausführungsformen der Erfindung dient im Zusammenhang mit der Zeichnung der näheren Erläuterung. Es zeigen:
- Figur 1:: eine Längsschnittansicht durch ein Bohrwerkzeug mit einer proximalen und einer distalen Schutzhülse bei Verwendung eines Bohrers in Form eines Spiralbohrers;
- Figur 2:: eine Ansicht ähnlich Figur 1 bei Verwendung eines Bohrers in Form eines Spickdrahtes bei in ein zu bohrendes Material vorgeschobenem Bohrer;
- Figur 3:: eine Schnittansicht längs Linie 3-3 in Figur 1 und
- Figur 4:: eine vergrößerte Detailansicht des Bereiches A in Figur 1.

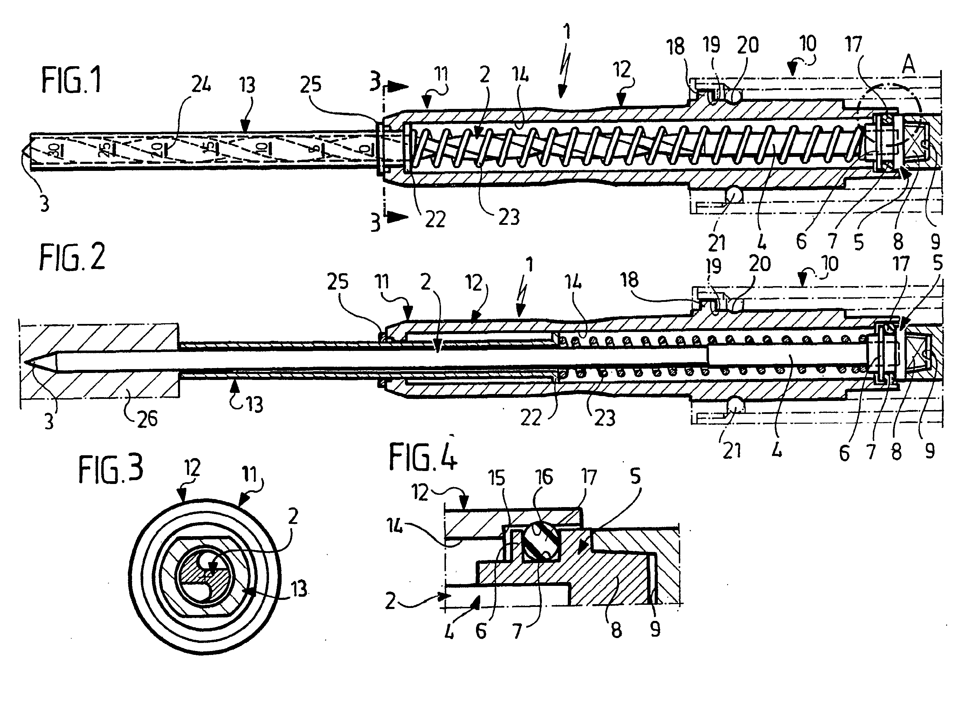

Das in der Zeichnung dargestellte Bohrwerkzeug 1 umfaßt im Ausführungsbeispiel der Figur 1 einen Bohrer 2 in Form eines Spiralbohrers, im Ausführungsbeispiel der Figur 2 in Form eines Spickdrahtes, ansonsten sind die Ausführungsbeispiele der Figuren 1 und 2 gleich.

An die Spitze 3 des Bohrers 2 an dessen distalem Ende schließt sich in proximaler Richtung ein Schaft 4 an, der an seinem proximalen Ende einen Bund 5 mit vergrößertem Außendurchmesser trägt. Dieser Bund kann einstückig mit dem Bohrer 2 ausgebildet sein oder er besteht aus einem getrennten Bauteil, das mit dem Schaft 4 fest verbunden ist. Beispielsweise kann der Bund 5 aus Kunststoff bestehen und durch Umspritzen des proximalen Endes des Schaftes 4 mit diesem verbunden werden.

Dieser Bund 5 umgibt das proximale Ende des Schaftes 4 hülsenförmig und bildet eine Ringstufe 6 aus, die durch eine Vergrößerung des Umfanges des Bundes 5 erzeugt wird. Unmittelbar neben dieser Ringstufe 6 ist in den Umfang des Bundes 5 eine Umfangsnut 7 eingearbeitet, in die ein elastischer O-Ring 17 eingelegt ist, der geringfügig über die Kontur der Umfangsnut 7 nach außen vorsteht.

Stirnseitig trägt der Bund 5 einen diametral verlaufenden, in proximaler Richtung abstehenden Mitnehmer 8, der ein Kupplungsteil bildet, welches formschlüssig in eine Kupplungsaufnahme 9 einschiebbar ist, die sich in einer in der Zeichnung nur strichpunktiert angedeuteten Bohrmaschine 10 befindet und die mittels eines in der Zeichnung nicht dargestellten Drehantriebs um die Längsachse des Bohrers 2 drehangetrieben ist.

Der Bohrer 2 wird umgeben von einem zweiteiligen Schutzgehäuse 11, welches eine proximale Schutzhülse 12 und eine distale Schutzhülse 13 umfaßt.

Die proximale Schutzhülse 12 ist im wesentlichen kreiszylindrisch aufgebaut und umgibt den Schaft 4 des Bohrers 2 im Abstand, so daß zwischen dem Schaft 4 und der Innenwand der proximale Schutzhülse 12 ein Ringraum 14 ausgebildet wird.

An ihrem proximalen Ende erweitert sich dieser Ringraum 14 an einer Stufe 15, der Durchmesser des Ringraumes 14 liegt dabei an der distalen Seite der Stufe 15 unterhalb dem Außendurchmesser der Ringstufe 6 des Bundes 5, auf der proximalen Seite der Stufe 15 dagegen geringfügig darüber.

Auf der Innenseite des Ringraumes 14 befindet sich auf der proximalen Seite der Stufe 15 eine flache Umfangsnut 16, die im dargestellten Ausführungsbeispiel einen kreisbogenförmigen Querschnitt aufweist. Beim axialen Aufschieben der proximalen Schutzhülse 12 auf den Bohrer 2 schnappt der in der Umfangsnut 7 eingelegte O-Ring 17 in die Umfangsnut 16 ein, so daß dadurch die proximale Schutzhülse 12 relativ zum Bohrer 2 in axialer Richtung festgelegt ist. Diese Festlegung kann durch Überwindung einer bestimmten Axialkraft gelöst werden, so daß es ohne weiteres möglich ist, den Bohrer 2 aus der proximalen Schutzhülse 12 herauszuziehen, wenn dies gewünscht wird.

Wenn die Umfangsnut 16 der Umfangsnut 7 gegenüberliegt, liegt auch die Stufe 15 dicht an der Ringstufe 6 an, die Stufe 15 bildet somit einen Anschlag aus, mit dem beim axialen Einschieben der proximalen Schutzhülse 12 in die Bohrmaschine 10 auch der Bohrer 2 in dieser Richtung mitgenommen wird, so daß der Mitnehmer 8 in die Kupplungsaufnahme 9 eintreten kann.

An der Außenseite trägt die proximale Schutzhülse 12 einen radial abstehenden Stift 18, der beim Einschieben der proximalen Schutzhülse 12 in die Bohrmaschine 10 in eine Axialnut 19 in der Bohrmaschine 10 eintritt und dadurch die proximale Schutzhülse 12 in der Bohrmaschine 10 gegen eine Verdrehung um deren Längsrichtung sichert.

Weiterhin weist die proximale Schutzhülse 12 auf ihrer Außenseite eine flache Umfangsnut 20 mit kreisbogenförmigem Querschnitt auf, in die ein kugelförmiger Verriegelungskörper 21 eintauchen kann, der in einer entsprechenden Führung der Bohrmaschine 10 in radialer Richtung verschiebbar gelagert ist. Dieser Verriegelungskörper 21 legt beim Eintauchen in die Umfangsnut 20 die proximale Schutzhülse 12 in axialer Richtung in der Bohrmaschine 10 fest, beim Zurückschieben des Verriegelungskörpers 21 wird hingegen die proximale Schutzhülse 12 freigegeben und kann dann in axialer Richtung aus der Bohrmaschine 10 herausgezogen werden.

Die proximale Schutzhülse erstreckt sich etwa über die halbe Länge des Bohrers 2. In die proximale Schutzhülse 12 ist die distale Schutzhülse 13 teleskopierend einschiebbar, sie umgibt den distalen Teil des Schaftes 4 des Bohrers 2 und reicht bei vollem Ausschieben aus dem Ringraum 14 bis zur Spitze 3 des Bohrers 2, wie dies in Figur 1 dargestellt ist. Durch eine bundförmige Erweiterung 22 wird verhindert, daß die distale Schutzhülse 13 vollständig aus der proximalen Schutzhülse 12 herausgezogen werden kann.

An dieser bundförmigen Erweiterung 22 stützt sich eine Schraubenfeder 23 ab, die den proximalen Teil des Schaftes 4 des Bohrers 2 umgibt und sich im Ringraum 14 befindet, das andere Ende der Schraubenfeder 23 ist an dem Bund 5 abgestützt, in diesem Bereich kann eine feste Verbindung zwischen Bund 5 und Schraubenfeder 23 vorliegen, beispielsweise kann das proximale Ende der Schraubenfeder 23 in den Bund 5 eingespritzt sein, wenn dieser aus einem Kunststoffspritzteil besteht.

Durch diese Schraubenfeder 23 wird die distale Schutzhülse 13 normalerweise vollständig aus der proximalen Schutzhülse 12 ausgeschoben, beim Einschieben der distalen Schutzhülse 13 in die proximale Schutzhülse 12 wird die Schraubenfeder 23 komprimiert.

Die distale Schutzhülse 13 hat einen kreisförmigen Querschnitt, der auf gegenüberliegenden Seiten abgeflacht ist, wie dies aus Figur 3 deutlich wird. Der Ringraum 14 ist am distalen Ende der proximalen Schutzhülse 12 mit einer im wesentlichen komplementären Öffnung versehen, so daß die distale Schutzhülse 13 relativ zur proximalen Schutzhülse 12 um deren Längsrichtung unverdrehbar, aber längsverschieblich geführt ist.

Auf der distalen Schutzhülse 13 ist eine Tiefenskala 24 aufgezeichnet, außerdem ist auf die distale Schutzhülse 13 ein Ring 25 aufgeschoben, der im Reibsitz auf der distalen Schutzhülse 13 gehalten ist, aber bei Überwindung bestimmter Reibungskräfte längs der distalen Schutzhülse 13 verschoben werden kann.

Der Bohrer 2 und die beiden ihn umgebenden Schutzhülsen 12 und 13 bilden gemeinsam das Bohrwerkzeug 1 aus, welches als Baueinheit ähnlich handhabbar ist wie ein herkömmlicher Bohrer. Diese Baueinheit kann mit einer Hand einfach dadurch mit der Bohrmaschine verbunden werden, daß die Baueinheit in axialer Richtung in die Bohrmaschine 10 eingeschoben wird, bis der Mitnehmer 8 in die Kupplungsaufnahme 9 eingreift. Dabei ist sichergestellt, daß der radial abstehende Stift der proximalen Schutzhülse 12 in die Axialnut 19 eintritt, so daß das Schutzgehäuse 11 drehfest mit der Bohrmaschine 10 verbunden wird. Eine axiale Festlegung des Schutzgehäuses 11 und damit auch des Bohrers 2 ergibt sich durch das Eintauchen der Verriegelungskörper 21 in die Umfangsnut 20.

Bei Bohrbeginn ist der Bohrer 2 über seine gesamte Länge allseits von dem Schutzgehäuse 11 umgeben. Beim Bohren dringt der Bohrer 2 mit seiner Spitze 3 in das zu bearbeitende Material 26 ein (Figur 2), dabei stützt sich die distale Schutzhülse 13 auf diesem Material 26 ab und wird beim Einschieben des Bohrers in das Material 26 ihrerseits entgegen der Wirkung der Schraubenfeder 23 in die proximale Schutzhülse 12 eingeschoben. Der außerhalb des Materials 26 angeordnete Teil des Bohrers 2 bleibt dabei dauerhaft nach außen abgeschirmt, so daß drehende Teile nicht mit dem umgebenden Gewebe in Verbindung kommen können.

Der Bohrer 2 und das Schutzgehäuse 11 können zu Reinigungszwekken sehr leicht voneinander getrennt werden, es genügt dazu, den Bohrer nach hinten aus dem Schutzgehäuse 11 herauszuziehen, sobald der O-Ring 17 aus der Umfangsnut 16 ausgeschnappt ist, sind beide Teile in axialer Richtung ohne weiteres zu trennen. Auf diese Weise ist es auch möglich, ein bestimmtes Schutzgehäuse 11 mit unterschiedlichen Bohrern 2 einzusetzen.

Die Eintauchtiefe des Bohrers 2 kann an der Tiefenskala 24 abgelesen werden, der Ring 25 wird beim Einschieben der distalen Schutzhülse 13 in die proximale Schutzhülse 12 von der letzteren auf der distalen Schutzhülse 12 entlang der Tiefenskala 24 verschoben und verbleibt in der am weitesten vorgeschobenen Stellung, so daß daran die maximale Eintauchtiefe ablesbar ist.

Das Schutzgehäuse 11 kann aus unterschiedlichen Werkstoffen bestehen, es können metallische Werkstoffe verwendet werden, es ist aber auch an sterilisierbare Kunststoffe zu denken.

Wenn der Bund 5 aus Kunststoff besteht, läßt sich eine Verbindung mit dem Schaft des Bohrers 2 auf die verschiedensten Weisen herstellen, erwähnt worden ist das Umspritzen des Schaftes, es ist auch eine Verklebung oder Verschweißung unter Einwirkung von Ultraschall möglich etc. Der Bund 5 kann auch aus Metall bestehen und mit dem Schaft verschweißt sein, es ist auch möglich, den Bund 5 einstückig mit dem Schaft 4 auszubilden.

## Patentansprüche

1. Bohrwerkzeug (1) für eine chirurgische Bohrmaschine (10) mit einem einen Schaft (4), eine Spitze (3) und ein Kupplungsteil zur Herstellung einer Drehverbindung mit einem Rotationsantrieb der Bohrmaschine (10) aufweisenden Bohrer (2), auf dessen Schaft (4) eine diesen umgebende proximale Schutzhülse (12) mit einem proximalen und einem distalen Ende gehalten ist, in weiche federnd eine den Bohrer (2) zwischen deren distalem Ende und der Spitze (3) des Bohrers (2) zumindest über einen Teil seiner Länge umgebende distale Schutzhülse (13) einschiebbar ist, **dadurch gekennzeichnet, daß** die proximale Schutzhülse (12) eine Drehsicherung (18, 19) aufweist, die bei in die Bohrmaschine (10) eingesetztem Bohrer (2) eine Drehung der proximalen Schutzhülse (12) relativ zur Bohrmaschine (10) verhindert.

2. Bohrwerkzeug nach Anspruch 1, **dadurch gekennzeichnet, daß** die distale Schutzhülse (13) im ausgeschobenen Zustand den Bohrer (2) bis über dessen Spitze (3) abdeckt.

3. Bohrwerkzeug nach einem der voranstehenden Ansprüche, **dadurch gekennzeichnet, daß** die proximale Schutzhülse (12) auf dem Schaft (4) des Bohrers (2) um dessen Längsachse verdrehbar gehalten ist.

4. Bohrwerkzeug nach Anspruch 3, **dadurch gekennzeichnet, daß** die proximale Schutzhülse (12) und der Schaft (4) des Bohrers (2) zueinander hin offene Umfangsnuten (7, 16) aufweisen, in die mindestens ein gemeinsames Lagerelement (17) eingreift.

5. Bohrwerkzeug nach Anspruch 4, **dadurch gekennzeichnet, daß** das Lagerelement (17) elastisch verformbar ist und beim axialen Verschieben der proximalen Schutzhülse (12) relativ zum Schaft (4) elastisch in eine der beiden Umfangsnuten (16) einschnappt.

6. Bohrwerkzeug nach Anspruch 5, **dadurch gekennzeichnet, daß** eine Umfangsnut (16) eine sehr geringe Tiefe aufweist und die andere Umfangsnut (7) eine größere Tiefe.

7. Bohrwerkzeug nach einem der Ansprüche 5 oder 6, **dadurch gekennzeichnet, daß** das Lagerelement (17) ein Ring ist.

8. Bohrwerkzeug nach einem der voranstehenden Ansprüche, **dadurch gekennzeichnet, daß** die Drehsicherung durch einen radialen Vorsprung (18) der proximalen Schutzhülse (12) gebildet wird, der in einen Rücksprung (19) der Bohrmaschine (10) eingreift.

9. Bohrwerkzeug nach einem der voranstehenden Ansprüche, **dadurch gekennzeichnet, daß** die proximale Schutzhülse (12) in der Bohrmaschine (10) in axialer Richtung festlegbar ist.

10. Bohrwerkzeug nach Anspruch 9, **dadurch gekennzeichnet, daß** die proximale Schutzhülse (12) mindestens einen Rücksprung (20) aufweist, in den ein Riegelvorsprung (21) der Bohrmaschine (10) einschiebbar ist.

11. Bohrwerkzeug nach einem der Ansprüche 9 oder 10, **dadurch gekennzeichnet, daß** die proximale Schutzhülse (12) einen Anschlag (15) trägt, mit dem sie eine Verschiebung des Bohrers (2) in distaler Richtung verhindert.

12. Bohrwerkzeug nach Anspruch 11, **dadurch gekennzeichnet, daß** das Kupplungsteil (8) in axialer Richtung formschlüssig in eine Kupplungsaufnahme (9) der Bohrmaschine (10) eingreift.

13. Bohrwerkzeug nach einem der voranstehenden Ansprüche, **dadurch gekennzeichnet, daß** der Bohrer (2) an seinem Schaft (4) einen Bund (5) trägt mit einer Kante (6), die an einer Stufe (15) der proximalen Schutzhülse (12) anliegt, der eine Umfangsnut (7) zur Aufnahme eines O-Ringes (17) aufweist, wobei der O-Ring (17) in eine gegenüberliegende Umfangsnut (16) der proximalen Schutzhülse (12) eingreift, und der stirnseitig einen Mitnehmer (8) trägt, der als Kupplungsteil in eine Mitnahmeöffnung (9) des Rotationsantriebes der Bohrmaschine (10) einführbar ist.

14. Bohrwerkzeug nach einem der voranstehenden Ansprüche, **dadurch gekennzeichnet, daß** der Schaft (4) von einer Schraubenfeder (23) umgeben ist, die sich einerseits am Schaft (4) und andererseits an der distalen Schutzhülse (13) abstützt.

15. Bohrwerkzeug nach Anspruch 14, **dadurch gekennzeichnet, daß** die Schraubenfeder (23) mindestens an einem Ende eine quer zu ihrer Längsachse verlaufende Endwindung aufweist, mit der sie an einer Abstützfläche des Schaftes beziehungsweise der distalen Schutzhülse (13) anliegt.

16. Bohrwerkzeug nach einem der voranstehenden Ansprüche, **dadurch gekennzeichnet, daß** die distale Schutzhülse (13) gegenüber der proximalen Schutzhülse (12) um deren Längsachse unverdrehbar ist.

17. Bohrwerkzeug nach Anspruch 16, **dadurch gekennzeichnet, daß** die distale Schutzhülse (13) einen unrunden Querschnitt aufweist und in eine komplementäre Öffnung der proximalen Schutzhülse (12) eintaucht.

18. Bohrwerkzeug nach einem der voranstehenden Ansprüche, **dadurch gekennzeichnet, daß** die distale Schutzhülse (13) eine Tiefenskala (24) trägt.

19. Bohrwerkzeug nach einem der voranstehenden Ansprüche, **dadurch gekennzeichnet, daß** auf der distalen Schutzhülse (13) ein Schleppring (25) angeordnet ist, der dort im Reibsitz gehalten und in axialer Richtung unter Überwindung der Reibkraft verschiebbar ist.

## Claims

1. A drilling tool (1) for a surgical drill (10), comprising a drill bit (2) having a shank (4), a tip (3) and a coupling part for establishing a rotary connection to a rotary drive of the drill (10), on which shank (4) is held a proximal protective sleeve (12) which has a proximal and a distal end and surrounds the shank (4) and into which a distal protective sleeve (13), surrounding the drill bit (2) between the distal end of the proximal protective sleeve (12) and the tip (3) of the drill bit (2) at least over part of its length, may be resiliently pushed, **characterised in that** the proximal protective sleeve (12) has an anti-rotation means (18, 19) preventing rotation of the proximal protective sleeve (12) relative to the drill (10) when the drill bit (2) is inserted into the drill (10).

2. A drilling tool according to claim 1, **characterised in that** the distal protective sleeve (13), when pushed out, covers the drill bit (2) as far as over its tip (3).

3. A drilling tool according to either one of the preceding claims, **characterised in that** the proximal protective sleeve (12) is held on the shank (4) of the drill bit (2) so as to be rotatable about the longitudinal axis thereof.

4. A drilling tool according to clam 3, **characterised in that** the proximal protective sleeve (12) and the shank (4) of the drill bit (2) have circumferential grooves (7, 16) which are open towards one another and in which at least one common bearing member (17) engages.

5. A drilling tool according to claim 4, **characterised in that** the bearing member (17) is resiliently deformable and snaps resiliently into one of the two circumferential grooves (16) when the proximal protective sleeve (12) is axially displaced relative to the shank (4).

6. A drilling tool according to claim 5, **characterised in that** one circumferential groove (16) has a very shallow depth and the other circumferential groove (7) has a greater depth.

7. A drilling tool according to either one of claims 5 and 6, **characterised in that** the bearing member (17) is a ring.

8. A drilling tool according to any one of the preceding claims, **characterised in that** the anti-rotation means is formed by a radial projection (18) of the proximal protective sleeve (12), the projection (18) engaging in a recess (19) of the drill (10).

9. A drilling tool according to any one of the preceding claims, **characterised in that** the proximal protective sleeve (12) is axially securable in the drill (10).

10. A drilling tool according to claim 9, **characterised in that** the proximal protective sleeve (12) has at least one recess (20) into which a locking projection (21) of the drill (10) is insertable.

11. A drilling tool according to either one of claims 9 and 10, **characterised in that** the proximal protective sleeve (12) carries a stop (15), by means of which it prevents displacement of the drill bit (2) in the distal direction.

12. A drilling tool according to claim 11, **characterised in that** the coupling part (8) positively engages in a coupling receiver (9) of the drill (10) in the axial direction.

13. A drilling tool according to any one of the preceding claims, **characterised in that** the drill bit (2) carries, on its shank (4), a collar (5) with an edge (6) which rests against a step (15) of the proximal protective sleeve (12), the collar (5) having a circumferential groove (7) for receiving an O-ring (17) which engages in an opposing circumferential groove (16) in the proximal protective sleeve (12), and at its end carrying a driver (8) which is insertable as a coupling part into a drive opening (9) in the rotary drive of the drill (10).

14. A drilling tool according to any one of the preceding claims, **characterised in that** the shank (4) is surrounded by a helical spring (23) supported at one end against the shank (4) and at the other end against the distal protective sleeve (13).

15. A drilling tool according to claim 14, **characterised in that** the helical spring (23) has an end turn which extends transversely to its longitudinal axis at least at one end and with which it rests against a supporting surface of the shank and/or the distal protective sleeve (13).

16. A drilling tool according to any one of the preceding claims, **characterised in that** the distal protective sleeve (13) is non-rotatable relative to the proximal protective sleeve (12) about the longitudinal axis thereof.

17. A drilling tool according to claim 16, **characterised in that** the distal protective sleeve (13) has a non-round cross-section and extends into a complementary opening in the proximal protective sleeve (12).

18. A drilling tool according to any one of the preceding claims, **characterised in that** the distal protective sleeve (13) carries a depth scale (24).

19. A drilling tool according to any one of the preceding claims, **characterised in that** an inertia ring (25) is arranged on the distal protective sleeve (13), where it is frictionally held, and is displaceable in the axial direction by overcoming the frictional force.

## Revendications

1. Outil de perçage (1) pour une perceuse (10) chirurgicale, comportant un foret (2) présentant un fût (4), une pointe (3) et une partie d'accouplement pour réaliser une liaison rotative avec un entraînement rotatif de la perceuse (10), sur le fût (4) duquel est retenu un manchon de protection proximal (12) entourant celui-ci, avec une extrémité proximale et une extrémité distale, manchon dans lequel peut être introduit de façon élastique un manchon de protection distal (13) entourant le foret (2) au moins sur une partie de sa longueur entre son extrémité distale et la pointe (3) du foret (2), **caractérisé en ce que** le manchon de protection proximal (12) présente une sécurité antirotation (18, 19) qui empêche une rotation du manchon de protection proximal (12) par rapport à la perceuse (10) lorsque le foret (2) est inséré dans la perceuse (10).

2. Outil de perçage selon la revendication 1, **caractérisé en ce que** le manchon de protection distal (13) recouvre le foret (2) jusqu'à sa pointe (3) lorsqu'il est à l'état extrait.

3. Outil de perçage selon l'une des revendications précédentes, **caractérisé en ce que** le manchon de protection proximal (12) est retenu sur le fût (4) du foret (2) de manière à tourner autour de son axe longitudinal.

4. Outil de perçage selon la revendication 3, **caractérisé en ce que** le manchon de protection proximal (12) et le fût (4) du foret (2) présentent des gorges périphériques (7, 16) ouvertes l'une vers l'autre, dans lesquelles s'engage au moins un élément de palier (17) commun.

5. Outil de perçage selon la revendication 4, **caractérisé en ce que** l'élément de palier (17) est élastiquement déformable et s'encliquette élastiquement dans l'une des deux gorges périphériques (16) lors du déplacement axial du manchon de protection proximal (12) par rapport au fût (4).

6. Outil de perçage selon la revendication 5, **caractérisé en ce qu'**une gorge périphérique (16) présente une profondeur très faible, et l'autre gorge périphérique (7) présente une profondeur plus importante.

7. Outil de perçage selon l'une ou l'autre des revendications 5 et 6, **caractérisé en ce que** l'élément de palier (17) est une bague.

8. Outil de perçage selon l'une des revendications précédentes, **caractérisé en ce que** la sécurité antirotation est formée par une saillie radiale (18) du manchon de protection proximal (12), saillie qui s'engage dans un retrait (19) de la perceuse (10).

9. Outil de perçage selon l'une des revendications précédentes, **caractérisé en ce que** le manchon de protection proximal (12) peut être immobilisé en direction axiale dans la perceuse (10).

10. Outil de perçage selon la revendication 9, **caractérisé en ce que** le manchon de protection proximal (12) présente au moins un retrait (20) dans lequel peut être introduite une saillie de verrou (21) de la perceuse (10).

11. Outil de perçage selon l'une ou l'autre des revendications 9 et 10, **caractérisé en ce que** le manchon de protection proximal (12) porte une butée (15) avec laquelle il empêche que le foret (2) se déplace en direction distale.

12. Outil de perçage selon la revendication 11, **caractérisé en ce que** la partie d'accouplement (8) s'engage en direction axiale par coopération de formes dans un logement d'accouplement (9) de la perceuse (10).

13. Outil de perçage selon l'une des revendications précédentes, **caractérisé en ce que** le foret (2) porte sur son fût (4) une collerette (5) avec une arête (6) qui prend appui sur un gradin (15) du manchon de protection proximal (12), collerette qui présente une gorge périphérique (7) pour recevoir un joint torique (17), le joint torique (17) s'engageant dans une gorge périphérique (16) opposée du manchon de protection proximal (12), et qui porte sur sa face frontale un toc d'entraînement (8) qui peut être introduit à titre de partie d'accouplement dans une ouverture d'entraînement (9) de l'entraînement rotatif de la perceuse (10).

14. Outil de perçage selon l'une des revendications précédentes, **caractérisé en ce que** le fût (4) est entouré par un ressort hélicoïdal (23) qui prend appui d'une part sur le fût (4) et d'autre part sur le manchon de protection distal (13).

15. Outil de perçage selon la revendication 14, **caractérisé en ce que** le ressort hélicoïdal (23) présente au moins à une extrémité une spire terminale s'étendant transversalement à son axe longitudinal, avec laquelle il repose sur une surface d'appui du fût ou du manchon de protection distal (13), respectivement.

16. Outil de perçage selon l'une des revendications précédentes, **caractérisé en ce que** le manchon de protection distal (13) ne peut pas tourner par rapport au manchon de protection proximal (12) autour de l'axe longitudinal de celui-ci.

17. Outil de perçage selon la revendication 16, **caractérisé en ce que** le manchon de protection distal (13) présente une section transversale non circulaire et plonge dans une ouverture complémentaire du manchon de protection proximal (12).

18. Outil de perçage selon l'une des revendications précédentes, **caractérisé en ce que** le manchon de protection distal (13) porte une graduation de profondeur (24).

19. Outil de perçage selon l'une des revendications précédentes, **caractérisé en ce que** sur le manchon de protection distal (13) est agencée une bague traînée (25) qui y est retenue par ajustement de friction et qui peut être déplacée en direction axiale en surmontant la force de friction.
